# EUROPEAN PATENT APPLICATION

(11) **EP 3 207 933 A1**
(43) Date of publication of application: **23.08.2017**
(21) Application number: 16156138.6
(22) Date of filing: 17.02.2016
(51) Int. Cl.: A61K 31/733, A61K 31/19, A61K 45/06, A61P 31/00, A61P 31/16, A61P 29/00

(54) **USES OF POLYFRUCTANS**

(71) Applicant: Proponent Biotech GmbH, 6301 Zug (CH)
(72) Inventor: ERNST, Bettina, 8032 Zürich (CH)
(74) Representative: Vossius & Partner Patentanwälte Rechtsanwälte mbB

(57) **Abstract**

The present invention relates to polyfructans and their uses, in particular medical uses for treating and/or attenuating and/or preventing viral infections, bacterial infections and/or inflammation. The invention furthermore relates to a composition comprising a polyfructan and, optionally, a short-chain fatty acid such as butyric acid, propionic acid or acetic acid.

## Description

The present invention relates to polyfructans and their uses, in particular medical uses for treating and/or attenuating and/or preventing viral infections, bacterial infections and/or inflammation. The invention furthermore relates to a composition comprising a polyfructan and, optionally, a short-chain fatty acid such as butyric acid, propionic acid or acetic acid.

The impact of infections upon individual's health and consequently the healthcare system and society as a whole is a worldwide issue. Current strategies to combat infections include vaccination and antibiotics, however these alone are not sufficient to control all infections and they increase the risk of selecting for pathogens that are resistant against these specific treatments.

Therefore, there is a need for means for use in treating and/or attenuating and/or preventing infections, in particular viral or bacterial infections, and/or inflammation.

The technical problem is solved by provision of the embodiments characterized in the claims.

Thus, the present invention relates to a polyfructan for use in treating and/or attenuating and/or preventing viral infections, bacterial infections and/or inflammation and compositions comprising the polyfructan.

That is, it is has been surprisingly and unexpectedly found by the present inventors that a diet rich in fermentable fibers, in particular a polyfructan, can protect against viral infections, bacterial infections and/or inflammation. As is demonstrated in the appended examples, mice were fed a fermentable fiber (inulin) in their diets, and this was compared to mice that were fed a non-fermentable fiber (cellulose) in their diets. Mice eating the inulin-rich diet were surprisingly protected against lethal infection by influenza virus. This was related to the mice having a lower viral load in their lungs and to reduced immunopathology caused by recruitment of neutrophils. An increased recruitment of CD8+ cytotoxic T cells likely aided in reducing the viral load, whilst an increased recruitment of CD4+ T regulatory cells likely aided in reducing immunopathology. The protective effect of the inulin diet could be reproduced by feeding butyrate to mice in their drinking water, which is a short-chain fatty acid that is produced in the gastrointestinal tract of mice eating an inulin-rich diet. Overall, the appended examples demonstrate that dietary consumption of fermentable fibers can protect against infections and tissue damage caused by inflammation. In light of this background, the present invention was made.

In a first aspect, the present invention relates to a polyfructan for use in treating and/or attenuating and/or preventing viral infections, bacterial infections and/or inflammation.

A polyfructan, within the meaning of the present invention, is a mixture of polysaccharides mainly consisting of fructose moieties, particularly D-fructose. D-fructose is a 6-carbon polyhydroxyketone, as shown below as Formula I. Crystalline fructose adopts a cyclic six-membered structure owing to the stability of its hemiketal and internal hydrogen-bonding. This form is formally called D-fructopyranose.

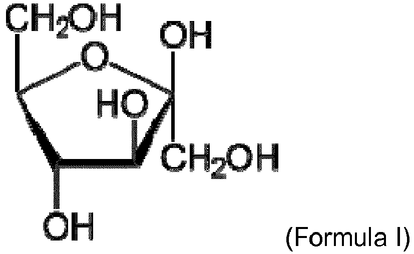

The fructose residues of the polysaccharides constituting the polyfructan are linked via glycosidic bonds. Within the present invention, a glycosidic bond or glycosidic linkage is a type of covalent bond that joins a carbohydrate (sugar) molecule to another carbohydrate. One distinguishes between α- and β-glycosidic bonds based on the relative stereochemistry (R or S) of the anomeric position and the stereocenter furthest from C1 in the saccharide. An α-glycosidic bond is formed when both carbons have the same stereochemistry, whereas a β-glycosidic bond occurs when the two carbons have different stereochemistry. In the present invention, the fructose residues of the polysaccharide comprised in the polyfructan are particularly linked via β-2,1-bonds. That is, particularly at least 50 mol-%, particularly at least 60 mol-%, 70 mol-%, 8 mol-%0, 90 mol-%, particularly all of the fructose residues comprised in the polyfructan are connected by β-2,1-linkages (as, e.g., in inulin).

The polysaccharides constituting the polyfructan of the present invention may, in addition to fructose, also comprise other ketohexoses such as psicose, sorbose and/or tagatose. The polysaccharides may also comprise aldohexoses such as glucose, allose, altrose, mannose, gulose, idose, galactose and/or talose. Together with fructose, the alternative sugar residues may build disaccharides comprised in the polysaccharide. For example, glucose and fructose build sucrose, which may also be comprised in the polysaccharide comprised in the mixture building the polyfructan used in the present invention. However, the main component of the polyfructan is fructose. Thus, in various embodiments of the invention about at least 70%, about at least 80%, about at least 90%, about at least 95% or almost all of the residues comprised in the polyfructan are fructose residues, particularly D-fructose residues.

While it is preferred that the major component of the polysaccharides is D-fructose, it is also preferred that the fructose residue at the 2-OH terminus of the polysaccharide chain(s) is bound, preferably via a β-2,1-linkage, to a glucose residue, preferably an α-D-glucose residue (Formula II).

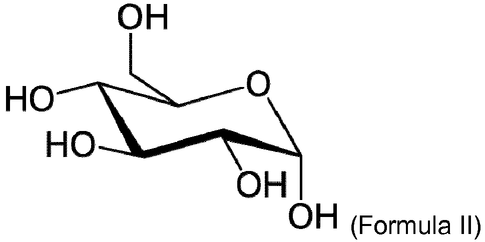

The polyfructan for use according to the present invention may comprise multiple polysaccharide chains, in particular chains of different lengths, i.e. with a different degree of polymerization. As such, the polyfructan may be a polydispersed mixture of polysaccharides of different length and of different sugar composition, as described above. Accordingly, provided herein is a polyfructan, wherein the polyfructan is a polydispersed mixture of polysaccharides having different degrees of polymerization, i.e. molecules of differing chain length. Preferably, the polysaccharides have a degree of polymerization between about 2 and about 60.

A preferred polyfructan for use in the present invention is inulin. Thus, the present invention, in one embodiment, relates to inulin for use in treating and/or attenuating and/or preventing viral infections, bacterial infections and/or inflammation.

Within the present invention, inulin is a heterogeneous collection of fructose polymers, i.e. polyfructans. Preferably, the inulin has chain-terminating glucosyl moieties and a repetitive fructosyl moiety, which are linked by β-(2,1) bonds, as shown below in Formula III (with n particularly between 2 and 60). The degree of polymerization (DP) preferably ranges from about 2 to about 60. It is preferred that the inulin has a degree of polymerization from about 10 to about 60. Because of the β(2,1) linkages, inulin is not digested by enzymes in the human alimentary system, contributing to its functional properties: reduced calorie value, dietary fiber and prebiotic effects. Without color and odor, it has little impact on sensory characteristics of food products.

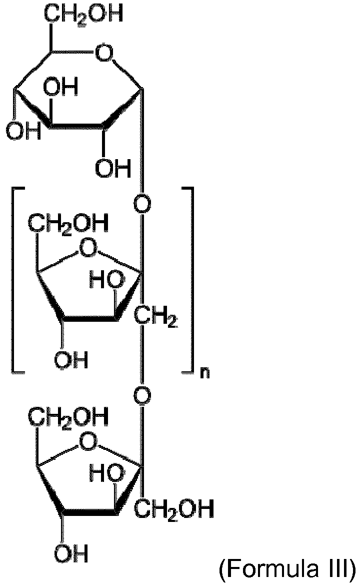

As described above, it is preferred that inulins used in the present invention are polymers composed mainly of fructose units, and having a terminal glucose. The fructose units are preferably joined by a β(2→1) glycosidic bond. Inulins are named in the following manner, where n is the number of fructose residues and py is the abbreviation for pyranosyl: Inulins with a terminal glucose are known as *alpha-D-glucopyranosyl-[beta-D-fructofuranosyl](n-1)-D-fructofuranosides,* abbreviated as GpyFn. Inulins without glucose are *beta-D-fructopyranosyl-[D-fructofuranosyl](n-1)-D-fructofuranosides,* abbreviated as FpyFn. While both inulin variants are provided herein, it is preferred that the inulin is a GpyFn.

The mainly linear chains of the polyfructan, in particular the inulin within the meaning of the invention, have, in one embodiment, one or more side chains essentially composed of fructosyl units, thus forming branched inulin molecules with a fructosyl-fructosyl linkage at the branching point commonly formed by a fructosyl- fructosyl β(2,6) bound. Accordingly, inulin molecules can be represented by the formula GFn or Fm wherein G represents a terminal glucosyl unit, F represents a fructosyl unit and n and m represent the number of fructosyl units linked to one another through a β(2,I) and/or a β(2,6) bound. The number n+1, respectively m, indicates the degree of polymerisation (DP) of the inulin molecule. Inulin is further characterised by its (number) average degree of polymerisation, represented by (DP). This is the value which corresponds to the total number of saccharide units (G and F units) in a given inulin sample divided by the total number of inulin molecules in said sample, without taking into account the monosaccharides glucose (G) and fructose (F) and the disaccharide sucrose (GF) which are possibly present in the sample. The average degree of polymerisation (DP) is commonly determined by the method described by De Leenheer L. et al., Starch/Stärke, 46 (5), 193-196, (1994) and Carbohydrates as Organic Raw Materials, Ed. H. Van Bekkum et al. for CRF, Wageningen, TheNetherlands, Vol. III, 67-74, (1996). Native inulin from plant sources (i.e. the inulin as present in the plant) appears as a polydisperse mixture of mainly linear polysaccharide chains with a (DP) ranging from 2 to about 100, whereas inulin molecules from bacterial origin, which commonly are branched ones, usually have much higher (DP) values, even up to about 115.000. Accordingly, it is preferred that the inulin provided herein is derived from natural plant sources with a DP ranging from about 2 to about 60, preferably from about 10 to about 60.

In this regard, the person skilled in the art is well-aware how to obtain inulin from natural plant sources. Inulin is commonly manufactured from plant sources, mainly from roots of Chicory (Cichorium intybus), but also from tubers of Jerusalem artichoke (Helianthus tuberosus) and from the pina (head) of the Blue Agave plant, in which inulin can be present in concentrations up to about 20 wt% on fresh plant material (hereinafter wt% means per cent by weight).

Inulin can be readily extracted from said plant parts and purified according to conventional techniques.

For example, artichoke roots may be used as starting material. Without being bound to the described process, inulin may be obtained from artichoke roots by first removing any adherent contaminants from the roots, e.g. by vigorous washing with water with a highpressure cleaner. It is advantageously possible to wash the roots in the deep-frozen state in order to minimize the loss of mass of root material. If necessary, the roots are initially comminuted coarsely, e.g. by chopping. Shredders are preferred for the further comminution. The product obtained is comminuted root material in the form of fibrous chips.

Preferably a ratio between the dry matter of the shredded material used and the water as extraction medium is established which leads to a dry matter content in the extract of 8 - 12 % by weight and an inulin content of more than 6 % by weight, preferably 6 - 8 % by weight, based on the weight of the extract. A correspondingly suitable choice of extraction conditions, such as the ratio of water to root weight, can lead to a transfer of 80 - 90 % by weight of the inulin present in the roots into the extract. The aforementioned conditions are suitable to achieve a favorable crystallization and a high yield of the inulin from the extract, based on the observation that the high molecular weight inulin crystallizes from the extract even at a concentration as low as 5% by weight, based on the weight of the extract.

There is no special restriction on the extraction equipment, and conventional extraction techniques for plant material can be applied. It is most preferred according to the invention for the extraction to take place in a jacket-heated extractor with agitator. In another highly preferred embodiment a heatable lauter tun is used as stirred extractor. Thus, the extraction of the inulin from the roots is combined with the separation of the extract from the spent chips by filtration, as described below. The extraction time after equilibration of the root/water mixture is preferably 30 min - 4 hours, preferably 1-2 hours. After this time, the extract is separated from the spent chips, e.g. by pumping off or straining off or filtration.

After separation of the extract from the spent chips, where appropriate, fibrous materials and plant fragments may remain as suspended materials in the extract. If present, these suspended materials are likewise removed from the extract. In this variant of the process, step b) of the process is thus followed, before step c), by a step in which suspended materials, mainly consisting of fibers, are removed from the extract. The acceptable amount of suspended materials and whether removal is to take place will be decided by the skilled worker from case to case. Removal of the suspended materials can take place by conventional separation techniques, as centrifugation or filtration. A desludging separator has proved particularly suitable. A screen or filter with appropriate fineness can also be used.

The suspended material can be filtered off by using the spent chips as a filter material. In this embodiment the spent chips are precipitated at the bottom of the extraction vessel equipped with a sieve at the bottom, like a lauter tun. The sieve is preferably a slit sieve. The precipitated spent chips are used as a filtration bed through which the extract flows. By using this technique a nearly quantitative removal of suspended material is possible without using further filtration steps before further refining or brightening the extract or crystallizing the inulin.

The extracts are colored owing to their content of coloring constituents and colloidally suspended colorized matter. The coloring constituents consist, inter alia, of tannins and flavanoids and usually confer a yellow or brownish yellow and/or dark brownish color on the extract. The inulins which can be obtained directly from such extracts do not comply with the desired requirements concerning a neutral color. It is therefore preferred to remove the coloring constituents from the extract in step c) of the process. Process step c) of the invention for removing coloring constituents from plant extracts is generally also referred to as decolorization, clarification or "brightening" of plant extracts. These terms are equivalent in the context of the present invention.

The whole of step c) of the process of the invention may if required also be carried out more than once.

After step c), inulin is precipitated from the extract in step d). The precipitation can be effected for example by adding alcohols such as ethanol, methanol or isopropanol. In this case, depending on the amount of alcohol added or adjusted polarity of the liquid phase, initially high molecular weight inulin fractions are precipitated, so that it is possible to influence, via the amount of alcohol added, how quantitatively the inulin present in the extract is precipitated and which molecular weight fractions are predominantly obtained. Besides alcohol, it is also possible to employ other nonpolar organic liquids which are miscible with water. For this purpose, in a particularly advantageous embodiment of this process step, to limit the use of alcohol, especially ethanol and isopropanol, the prepared extract is initially concentrated, preferably to one fourth to one fifth of its initial volume. The concentration can take place by evaporation or membrane filtration and a combination of both processes. Care must be taken in this case that the concentrate is kept hot during the concentration, preferably at 60-95°C, in order to avoid precipitation of the inulin. An advantage of membrane filtration is the depletion, associated therewith, in low molecular weight substances accompanying the inulin. The subsequent precipitation of the inulin from the concentrate can be managed by the choice of increasing alcohol concentration so that the inulin is fractionated according to molecular size ranges which are characterized for example by the weight average degree of polymerization (DPw). Depending on the choice of the precipitation conditions, the result is fractions which have the DPw according to the invention.

It is more preferred to obtain inulin by cooling the extract than by alcoholic precipitation. The preferred conditions are such that the extract is cooled to a temperature of 2 - 10°C, more preferably 2 - 8°C, and kept at this temperature over a period of from 6 to 140 h, preferably 6 to 48 h, during which the inulin precipitates. The cooling rate and temperature, and the duration of the cooling influence the precipitation of the inulin from the extract and the breadth of the molecular weight distribution and thus at the same time the quantity. Choice of a longer period and lower temperature results in precipitation of more low molecular weight inulins and a broader molecular weight distribution and thus a lower average molecular weight of the precipitated fraction. The precipitated inulin is separated from the liquid phase by conventional separation techniques such as, for example, centrifugation, decantation, filtration.

In a preferred embodiment, inulin is crystallized for the first time after the extraction step b) and before step c) of the above described process. Such crystallisation is preferably done as described previously. Crystallisation before step c) leads to an increase in the yield of high molecular weight inulin compared with direct brightening of the extract, and economizes the use of the brightening agents, i.e. magnesium compound and the alkaline component. It is advantageous to brighten the extract after the first crystallisation of the inulin as in this case only the coloring constituents bound to the inulin crystals have to be removed, which leads to a similarly smaller amount of inulin bound to the brightening sludge. A first precipitation and removal of the precipitated inulin can be followed by renewed cooling of the extract or addition of alcohol in order to obtain any inulin fractions which are still dissolved. A decision about repetition is made from case to case according to how quantitatively the inulin is to be obtained from the plants and what molecular weight distribution in the final product is desired.

The inulin concentration in the extract depends substantially on the inulin content of the roots and the concentration of the comminuted roots in the extract and is a further variable which has an effect on the precipitation of the inulin by cooling the extract. The dependence of the precipitation on the concentration can therefore be utilized in order to concentrate the liquid phase after the first precipitation, e.g. by evaporation, in order also to precipitate the low molecular weight fractions if this is desired.

In the last process step e), the precipitated inulin is reprecipitated. "Reprecipitation" means in the context of this invention that the solid inulin, resulting from the previous process step, is redissolved and then precipitated and/or crystallized out of the solution again. Thus, process step e) can also be worded as: the inulin is dissolved and precipitated and/or crystallized again, wherein this step is done at least once. The crystallization differs from the precipitation in that predominantly crystalline structures are obtained.

The inulin is preferably dissolved under the influence of heat and preferably in water. Water with a temperature of 70-100°C, in particular 90-100°C, is particularly suitable.

The precipitation in step e) can take place by alcoholic precipitation as previously described. However, the inulin is preferably obtained by cooling the solution to 2 - 10°C, more preferably 2-8°C, over a period of 6 to 140 h, preferably 6 to 48 h.

The precipitation of the inulin dissolved in step e) can be repeated in order to obtain the inulin still remaining in the liquid phase. A decision about repetition is to be made from case to case according to how quantitatively the inulin is to be obtained from the plants and what molecular weight distribution in the final product is desired. The liquid phase can be concentrated in order to simplify the precipitation. After reprecipitation, the resulting inulin solid is separated from the liquid phase by conventional separation techniques such as, for example, centrifugation, decantation, filtration.

In order to influence the molecular mass distribution and purity of the resulting inulin product, process step e) can be carried out more than once. It has emerged that the averages of the molecular weight and the averages of the degree of polymerization are shifted to higher values on repetition of the reprecipitation step e). It is thus possible to set various averages of the molecular weight/degree of polymerization of the inulin of the invention within the claimed range.

If fine-particle impurities are still present, it is advantageous to insert one or more filtration steps into the process. Any fine-particle impurities present are removed in the filtration. The fineness of the filter is chosen by the skilled worker depending on the particle size of the impurity.

The filtration step(s) can be inserted anywhere in the process after obtaining the extract. A filtration step directly after obtaining the extract in step b) for example is advantageous. The filtration step is to be distinguished from the removal of suspended materials as described previously, because the particles removed by the filtration are finer than the suspended materials, which consist mainly of fibers. In a further preferred embodiment, the filtration step is carried out before step d).

The filtration step is preferably combined with a reprecipitation as described for process step e). This entails the inulin being dissolved as previously described for step e), and the solution then being filtered. After the filtration, the inulin is precipitated or crystallized out of the filtered solution. The solid inulin resulting after the precipitation or crystallization can be separated from the liquid phase by conventional separation techniques, such as, for example, centrifugation, decantation and filtration.

The resulting inulin is preferably dried in a further, last process step. The drying can take place by freeze drying, spray drying or drum drying.

In a preferred embodiment, the inulin of the invention is in spray-dried form. Suitable spray- drying parameters are described in the appended examples. It is self evident that in case of a spray drying process a precipitated or crystallized inulin must be brought into suspension (in water below about 80°C) or into solution (in water above about 80°C) again. Alternatively, a last precipitation or crystallization step, as described above, can be omitted and the suspended or dissolved inulin from the process can directly be spray dried. It is possible by adding spray-dried inulins of the invention to liquid prepared food products for the viscosity to be increased particularly effectively. On addition of equal quantities of inulin of the invention, a greater increase in viscosity is achieved with a spray-dried inulin compared with an inulin dried in another way (e.g. freeze drying).

In yet a further preferred embodiment, the inulin of the invention is in spray-granulated form. Spray-granulated inulin is obtained by known processes, e.g. by introducing a previously spray- dried material as granulation seed and spray drying further inulin. An inulin with a particle size of 10-100 µm for example can serve as initial charge. Suitable spray-granulation conditions are for example a feed composition of 70% water and 30% inulin and a feed temperature of 90°C.

Natural inulin from chicory, respectively from J. artichoke, commonly appears as a polydisperse mixture of slightly branched chains (typically chains with less than 2 per cent, respectively less than 1 per cent, branching) with a (DP) ranging from 2 to about 60, respectively from 2 to about 40.

Natural inulin from agave appears as a polydisperse mixture of highly branched chains with a (DP) commonly ranging from about 14 to about 17. At industrial scale, chicory inulin is conventionally obtained by extraction of shredded chicory roots with hot water yielding a crude inulin solution which is subsequently purified by depuration (treatment with lime followed by carbonatation and filtration) and by refining (involving treatment over ion-exchangers, treatment with active carbon and filtration). Standard grade inulin is then commonly obtained from the purified and refined solution by spray-drying. Optionally, monomeric and dimeric saccharides are removed from the purified and refined solution (e.g. by column chromatographic separation as described in EP 0 670 850) to yield via spray-drying an inulin grade with a standard (DP) of about 10 which is about free of monomeric and dimeric saccharides. Optionally the purified and refined solution can be fractionated to remove monomeric and dimeric saccharides as well as oligofructose (e.g. by directed crystallisation as described in EP 0 769 026) and the fractionated inulin is then isolated in particulate form by spray-drying. Depending on the manufacturing process, chicory inulin with a (DP) ranging from about 10 (standard grade) to about 30, and even more, can be obtained.

Similarly, agave inulin can be obtained at industrial scale by squeezing, or extracting with water, shredded heads or pulp from Blue Agave, followed by conventional purification, refining and isolation of the inulin e.g. via spray-drying.

Inulin from chicory is for example commercially available as RAFTILINE® from ORAFTI (Tienen, Belgium) in various grades. Typical grades are RAFTILINE® ST (with a (DP) of about 10 and containing in total about 8 % by weight glucose, fructose and sucrose) and RAFTILINE® HP (with a (DP) of at least 20, commonly with a (DP) of about 23 to about 25, and virtually free of glucose, fructose and sucrose).

Agave inulin is commercially available, for example industrial grade agave inulin as GAVEDIET® PR with a (DP) of 14 -16 and containing in total about 5 % by weight of glucose and fructose, from Industrias Colibri Azul S.A. de C.V., Mexico.

Accordingly, provided herein is a polyfructan for use in treating and/or attenuating and/or preventing viral infections, bacterial infections and/or inflammation, wherein the polyfructan is inulin and wherein the inulin is derived from plants, preferably the roots of plants (or the head of the agave plant). It is preferred that the plant is of the genus *Cynara, Cichorium, Helianthus, Dahlia, Agave* or *Taraxacum.* It is furthermore preferred that the plant of the genus *Cynara* is *Cynara cardunculus* (cardoon) or *Cynara scolymus* (artichoke). It is also preferred that the plant of the genus *Cichorium* is *Cichorium intybus* (chicory). Furthermore, it is preferred that the plant of the genus *Helianthus* is *Helianthus tuberosus* (Jerusalem archichoke). Finally, it is preferred that the plant of the genus *Agave* is *Agave tequilana* (Blue agave).

Accordingly, the present invention provides in one embodiment a polyfructan, particularly a polyfructan comprising a β-2,1-linked chain of fructose residues, for use in treating and/or attenuating and/or preventing viral infections, bacterial infections and/or inflammation.

In one embodiment, the fructose residue at the 2-OH terminus of said β-2,1-linked chain is bound, particularly via a β-2,1-linkage, to a glucose residue.

In one embodiment, the polyfructan for use according to the present inveniton as described herein in the various embodiments is a polydispersed mixture of molecules of differing chain length.

In a specific embodiment of the invention, the polyfructan for use according to the present inveniton as described herein in the various embodiments is inulin, particularly inulin, which has a degree of polymerization of between about 2 to about 60, particularly of between about 10 to about 60.

In another specific embodiment, said inulin is derived from a plant, particularly a plant of the genus *Cynara,* particularly *Cynara cardunculus* (cardoon) or *Cynara scolymus* (artichoke), *Cichorium,* particularly *Cichorium intybus* (chicory), *Helianthus,* particularly *Helianthus tuberosus* (Jerusalem archichoke), *Dahlia, Agave,* particularly *Agave tequilana* (Blue agave) or *Taraxacu,* particularly from the roots of the plant.

In a further embodiment, the present invention relates to a composition comprising the polyfructan, preferably the inulin, as described herein in the various embodiments for use in treating and/or attenuating and/or preventing viral infections, bacterial infections and/or inflammation. In a specific embodiment, the polyfructan content of the composition is between about 5 to about 60, preferably about 10 to about 50, about 20 to about 40%, or about 30% (w/w). The composition may further comprise a short-chain fatty acid such as butyric acid, propionic acid or acetic acid.

In one embodiment of the invention, the present invention relates to the polyfructan, preferably the inulin, as described herein in the various embodiments, or to the composition comprising the polyfructan, preferably the inulin, as described herein in the various embodiments, for use in protecting a subject against lung dysfunction upon administration of said polyfructan or composition of the invention to said subject, particularly when compared to a control subject, which had not received said polyfructan or composition of the invention. In a specific embodiment, the lung dysfunction is determined by measuring the airway resistance by airflow perturbations using the FlexiVent invasive airway mechanics system from Scireq as shown in Example 1.

In another specific embodiment, the subject to be treated is fed a diet high in polyfructan fibers, particularly a diet high in inulin, particularly a diet containing 30% inulin and the control subject is fed a diet high in a non-fermentable fibers, particularly a diet high in cellulose, particularly a diet containing 30% cellulose.

In one embodiment of the invention, the present invention relates to the polyfructan, preferably the inulin, as described herein in the various embodiments, or to the composition comprising the polyfructan, preferably the inulin, as described herein in the various embodiments, for use in protecting a subject against lung pathology and vascular leakage caused by influenza virus upon administration of said polyfructan or composition of the invention to said subject, particularly when compared to a control subject, which had not received said polyfructan orcomposition of the invention.

In a specific embodiment, lung pathology and vascular leakage is determined by standard histological processing and staining of a lung tissue sample with hematoxylin and eosin stain as shown in Example 3.

In another specific embodiment, the subject to be treated is fed a diet high in polyfructan fibers, particularly a diet high in inulin, particularly a diet containing 30% inulin and the control subject is fed a diet high in a non-fermentable fibers, particularly a diet high in cellulose, particularly a diet containing 30% cellulose.

In one embodiment of the invention, the present invention relates to the polyfructan, preferably the inulin, as described herein in the various embodiments, or to the composition comprising the polyfructan, preferably the inulin, as described herein in the various embodiments, for use in protecting a subject against the infiltration of neutrophils into the airways following influenza virus infection upon administration of said polyfructan or composition of the invention to said subject, particularly when compared to a control subject, which had not received said polyfructan or composition of the invention.

In a specific embodiment, the cells are examined and based upon standard morphological and cytochemical criteria are determined to be eosinophils, macrophages, neutrophils of lymphocytes. Cells are stained with antibodies against CD4, CD8, CD107, Granzyme B (Biolegend), and tetramer (TCMetrix), Foxp3 and acquired by flow cytometry using a BD LSR II followed by analysis with Flowjo (version 10.0.8) as shown in Example 4.

In another specific embodiment, the subject to be treated is fed a diet high in polyfructan fibers, particularly a diet high in inulin, particularly a diet containing 30% inulin and the control subject is fed a diet high in a non-fermentable fibers, particularly a diet high in cellulose, particularly a diet containing 30% cellulose.

In one embodiment of the invention, the present invention relates to the polyfructan, preferably the inulin, as described herein in the various embodiments, or to the composition comprising the polyfructan, preferably the inulin, as described herein in the various embodiments, for use in increasing in a subject recruitment of lymphocytes, of which a higher proportion of cells are highly activated cytotoxic T cells specific to the influenza virus and T regulatory cells, into the airways following influenza virus infection, upon administration of said polyfructan or composition of the invention to said subject, particularly when compared to a control subject, which had not received said polyfructan or composition of the invention.

In another specific embodiment, the subject to be treated is fed a diet high in polyfructan fibers, particularly a diet high in inulin, particularly a diet containing 30% inulin and the control subject is fed a diet high in a non-fermentable fibers, particularly a diet high in cellulose, particularly a diet containing 30% cellulose.

For treating and/or attenuating and/or preventing viral infections, bacterial infections and/or inflammation, the polyfructan or the composition of the invention may be administered orally, sublingually, topically, intranasally, inhaled, parenterally, intravenously, intramuscularly or subcutaneously.

In one embodiment, the present invention relates to the polyfructan, preferably the inulin, as described herein in the various embodiments, or to the composition comprising the polyfructan, preferably the inulin, as described herein in the various embodiments, for use in reducing the virus load in lung tissue, particularly influenza virus load in lung tissue, upon administration of said polyfructan or composition of the invention to said subject, particularly when compared to a control subject, which had not received said polyfructan or composition of the invention.

In a specific embodiment, the cells are examined using RNA extraction from homogenized lung tissue and PCR amplification of viral nucleic acids as shown in Example 5.

In another specific embodiment, the subject to be treated is fed a diet high in polyfructan fibers, particularly a diet high in inulin, particularly a diet containing 30% inulin and the control subject is fed a diet high in a non-fermentable fibers, particularly a diet high in cellulose, particularly a diet containing 30% cellulose.

As provided herein, the polyfructan, preferably the inulin, of the invention is used for treating and/or attenuating and/or preventing viral infections. In this regard, a viral infection occurs when an organism's body is invaded by pathogenic viruses, and infectious virus particles (virions) attach to and enter susceptible cells. A virus is a small parasite consisting of nucleic acid (RNA or DNA) enclosed in a protein coat. Viruses can only replicate by infecting a susceptible host cell and directing the host cell machinery to produce more viruses. Glycoproteins (located in the protein coat) mediate the adsorption to, and the penetration of, the virus into susceptible host cells.

Most viruses are classified into broad categories based on the types of nucleic acids formed during replication and the pathway by which mRNA is produced. In general, viruses have either RNA or DNA as their genetic material, wherein the nucleic acid can be single- or double-stranded.

Important virus families of the DNA type (also classified as Classes I and II viruses-See Harvey, L. et al., Molecular Cell Biology, Fourth Edition, W. H. Freeman and Company (2000)) include adenoviridae, herpesviridae, poxviridae, papovaviridae, densovirinae, and parvovirinae. Virus families typically classified of the RNA type (also classified as Classes III-VI, See *Molecular Cell Biology)* include bimaviridae, reoviridae, astoviridae, arterivirus, caliciviridae, coronaviridae, flaviviridae, picomaviridae, togaviridae, polioviruses, bomaviridae, filoviridae, paramyxovirinae, pneumovirinae, rhabdoviridae, bunyaviridae, and orthomyxoviridae.

Influenza, commonly known as the "flu," is a contagious disease that is caused by the influenza virus, classified in the orthomyxoviridae family. There are three known influenza-type viruses which affect human beings: Influenza A, B and C. Influenza A viruses have been isolated from many animal species in addition to humans, while the influenza B and C viruses have been found to infect mainly humans. Without being limited thereto, the means of the present invention are particularly useful for treating and/or attenuating and/or preventing an infection with an influenza causing virus.

Influenza viruses are enveloped viruses containing negative single-stranded RNA's which are segmented and encapsidated. The influenza virus envelope is characterized by the presence of two surface glycoproteins: hemagglutinin and neuraminidase. The influenza A and B virions are pleomorphic and are usually 80-120 nm in diameter. The influenza C virion has many distinctive properties and is thus distinguished from the closely related A and B virions.

Influenza viruses attack the respiratory tract in humans (i.e., nose, throat, and lungs). For example, infection with influenza A or B often can cause a highly contagious, acute respiratory illness. Influenza infection usually includes the following symptoms: fever, headache, tiredness (can be extreme), dry cough, sore throat, nasal congestion, and body aches.

Accordingly, the present invention provides for the treatment and/or prevention of viral infections from Classes I through V viruses (see Lodish, H. et al., Molecular Cell Biology, Fourth Edition, W. H. Freeman and Company (2000)) through the administration of the polyfructan, preferably the inulin of the invention, to a subject. More specifically, the present invention provides methods for the treatment and/or prevention of a Class I-V viral infection; the alleviation of Class I-V viral infection-related symptoms; as well as the prevention or delay in development of Class I-V viral infection-related complications.

Viral infections resulting from the following types of viruses are treated and/or prevented by administering a polyfructan, preferably inulin, as disclosed herein. The viruses include double-stranded DNA (dsDNA), single-stranded DNA (ssDNA), double-stranded genomic RNA (dsRNA), single-strand positive RNA, and single-strand negative RNA viruses, such as but not limited to, influenza viruses, adenoviruses; herpesviruses; human papillomaviruses; parvoviruses; reoviruses; picomaviruses; coronaviruses; flavivirus; togaviruses, orthomyxovirus; bunyaviruses; rhabdoviruses; and paramyxoviruses.

The present invention is particularly applicable to both human and animal health.

Specifically exemplified herein is the use of a polyfructan, preferably inulin, to treat and/or prevent an influenza virus infection. In accordance with the subject invention, administration of a polyfructan, preferably inulin, to a subject prior to acquiring the influenza virus can help protect the subject from influenza infection, or at least ensure that symptoms related to influenza virus disease develop to a lesser extent than would be observed in the absence of the cysteamine compound.

In another embodiment, a polyfructan, preferably inulin, is administered to prevent and/or delay the development of influenza-related complications in subjects who are at an increased risk of contracting those complications. For example, influenza-related complications such as encephalitis, bronchitis, tracheitis, myositis rhinitis, sinusitis, asthma, bacterial infections (i.e., streptococcus aureus bacterial infection, haemophilus influenzae bacterial infection, staphylococcal pneumonia bacterial infection), cardiac complications (i.e., atrial fibrillation, myocarditis, pericarditis), Reye's syndrome, neurologic complications (i.e., confusion, convulsions, psychosis, neuritis, Guillain-Barre syndrome, coma, transverse myelitis, encephalitis, encephalomyelitis), toxic shock syndrome, myositis, myoglobinuria, and renal failure, croup, otitis media, viral infections (i.e., viral pneumonia), pulmonary fibrosis, obliterative bronchiolitis, bronchiectasis, exacerbations of asthma, exacerbations of chronic obstructive pulmonary disease, lung abscess, empyema, pulmonary aspergillosis, myositis and myoglobinaemia, heart failure, early and late fetal deaths in pregnant women, increased perinatal mortality in pregnant women, congenital abnormalities in birth, can be reduced through consumption, according to the subject invention, of a polyfructan, preferably inulin.

The polyfructan, preferably inulin, may also be used in the treatment and/or prevention of bacterial infections. Without being limited threeto, a bacterial pathogen may be derived from a bacterial species selected from the group consisting of: *Staphylococcus* spp., e.g. *Staphylococcus aureus, Staphylococcus epidermidis; Enterococcus* spp., e.g. *Enterococcus faecalis; Streptococcus pyogenes; Listeria* spp.; *Pseudomonas* spp.; *Mycobacterium* spp., e.g. *Mycobacterium tuberculosis; Enterobacter* spp.; *Campylobacter* spp.; *Salmonella* spp.; *Streptococcus* spp., e.g. *Streptococcus* Group A or B, *Streptoccocus pneumoniae; Helicobacter* spp., e.g. *Helicobacter pylori, Neisseria* spp., e.g. *Neisseria gonorrhea, Neisseria meningitidis; Borrelia burgdorferi; Shigella* spp., e.g. *Shigella flexneri; Escherichia coli; Haemophilus* spp., e.g. *Haemophilus influenzae; Chlamydia* spp., e.g. *Chlamydia trachomatis, Chlamydia pneumoniae, Chlamydia psittaci; Francisella tularensis; Bacillus* spp., e.g. *Bacillus anthracis; Clostridia* spp., e.g. *Clostridium botulinum; Yersinia* spp., e.g. *Yersinia pestis; Treponema* spp.; and *Burkholderia* spp.; e.g. *Burkholderia mallei* and *Burkholderia pseudomallei.*

The polyfructan, preferably inulin, may also be used in the treatment and/or prevention of inflammation. Inflammation is part of the complex biological response of body tissues to harmful stimuli, such as pathogens, damaged cells, or irritants. Inflammation is a protective response that involves immune cells, blood vessels, and molecular mediators. The purpose of inflammation is to eliminate the initial cause of cell injury, clear out necrotic cells and tissues damaged from the original insult and the inflammatory process, and to initiate tissue repair. The classical signs of acute inflammation are pain, heat, redness, swelling, and loss of function. Inflammation is a generic response, and therefore it is considered as a mechanism of innate immunity, as compared to adaptive immunity, which is specific for each pathogen. Inflammation can be classified as either *acute* or *chronic. Acute inflammation* is the initial response of the body to harmful stimuli and is achieved by the increased movement of plasma and leukocytes (especially granulocytes) from the blood into the injured tissues. A series of biochemical events propagates and matures the inflammatory response, involving the local vascular system, the immune system, and various cells within the injured tissue. Prolonged inflammation, known as *chronic inflammation,* leads to a progressive shift in the type of cells present at the site of inflammation, such as mononuclear cells, and is characterized by simultaneous destruction and healing of the tissue from the inflammatory process.

The present invention furthermore relates to a foodstuff comprising a polyfructan, preferably inulin. The foodstuff may be selected from dairy products, yoghurts, ice creams, milk-based soft ice, milk-based garnishes, puddings, milkshakes, egg custard, cheeses, nutrition bars, energy bars, breakfast bars, confectionery, bakery products, crackers, cookies, biscuits, cereal chips, snack products, ice tea, soft ice made from fruit juice, diet drinks, finished drinks, sports drinks, stamina drinks, powdered drink mixtures for dietary supplementation, infant and baby food, calcium-supplemented orange juice, bread, croissants, breakfast cereals, noodles, spreads, sugar-free biscuits and chocolates, calcium chews, meat products, mayonnaise, salad dressings, nut butter, deep-frozen meals, sauces, soups and ready-to-serve meals. As such, the dietary products provided above may be used to treat and/or prevent a viral infection, bacterial infection and/or inflammation.

Furthermore, provide herein is a dietary supplement comprising a polyfructan, preferably inulin. The supplement may be used in any of the above foodstuffs.

For the above described applications, the polyfructan, preferably inulin, may be prepared as aqueous paste. The aqueous paste is obtainable by dispersing the polyfructan, preferably inulin in water, shearing the resulting dispersion until homogeneous, storing the product obtained in this way at 4-15°C for 12-24 h and, after conditioning to room temperature, stirring to give a homogeneous paste. A preferred paste comprises water and 1-40% by weight, more preferably 1 - 35 % by weight, still more preferably 1 - 30 % by weight, even more preferably 2 - 25 % by weight, yet more preferably 2 - 20 % by weight, and particularly preferably 10-20% by weight inulin based on the total weight of the paste. The term "paste" is according to this invention equivalent to a suspension of cristalline and/or amorphous inulin. Accordingly, the term "aqueous paste" is to be understood as a suspension of cristalline and/or amorphous inulin in aqueous phase. The aqueous phase is based on water which can optionally comprise further dissolved or suspended substances, such as salts, other carbohydrates, proteins, amino acids. In an advantageous embodiment the inulin in the paste is is a spray dried inulin, i.e. an inulin which was spray dried before forming the paste.

A preferred level of the polyfructan, preferably inulin in foodstuffs, especially in dairy, particularly in yoghurt, is 0,2 - 5 % by weight, preferably 0,5 - 4,5 % by weight of dry inulin, based on the total weight of all components of the foodstuff, dairy, or yoghurt.

The technical terms and expressions used within the scope of this application are generally to be given the meaning commonly applied to them in the pertinent art if not otherwise indicated herein below.

As used in this specification and the appended claims, the singular forms "a", "an", and "the" include plural referents unless the context clearly dictates otherwise. Thus, for example, reference to "a compound" includes one or more compounds.

The terms "treatment", "treating" and the like are used herein to generally mean obtaining a desired pharmacological and/or physiological effect. The effect may be prophylactic in terms of completely or partially preventing a disease or symptom thereof and/or may be therapeutic in terms of partially or completely curing a disease and/or adverse effect attributed to the disease. The term "treatment" as used herein covers any treatment of a disease in a subject and includes: (a) preventing a disease related to an undesired immune response from occurring in a subject which may be predisposed to the disease; (b) inhibiting the disease, i.e. arresting its development; or (c) relieving the disease, i.e. causing regression of the disease.

A "patient" or "subject" for the purposes of the present invention is used interchangeably and meant to include both humans and other animals, particularly mammals, and other organisms. Thus, the methods are applicable to both human therapy and veterinary applications. In the preferred embodiment the patient or subject is a mammal, and in the most preferred embodiment the patient or subject is a human.

The term "attenuation" as used herein refers to reduction of a viral infection in a subject or in a tissue of a subject, particularly in lung tissue of a subject, i.e. reduction or clearance of the amount of virus or viral load. The particular degree or level of the reduction or clearance is at least 15%, 25%, 35%, 50%, 65%, 75%, 80%, 85%, 90%, 95%, 98% or more.

The terms "myeloid precursors", "myeloid lineage" or "myeloid cells" refer all to multipotent stem cells as one of two lineages of hematopoietic cells, which are able to develop into monocytes, macrophages, dendritic cells, neutrophils, eosinophils, basophils, megacaryocytes, platelets or erythrocytes.

The expressions "pharmaceutical composition" and "therapeutical composition" are used herein interchangeably in the widest sense. They are meant to refer, for the purposes of the present invention, to a therapeutically effective amount of the active ingredient, i.e. the inulin or a pharmaceutically acceptable salt thereof, optionally, together with a pharmaceutically acceptable carrier or diluent.

It embraces compositions that are suitable for the curative treatment, the control, the amelioration, an improvement of the condition or the prevention of a disease or disorder in a human being or a non-human animal. Thus, it embraces pharmaceutical compositions for the use in the area of human or veterinary medicine. Such a "therapeutic composition" is characterized in that it embraces at least one inulin compound or a physiologically acceptable salt thereof, and optionally a carrier or excipient whereby the salt and the carrier and excipient are tolerated by the target organism that is treated therewith.

A "therapeutically effective amount" refers to that amount which provides a therapeutic effect for a given condition and administration regimen. In particular, "therapeutically effective amount" means an amount that is effective to prevent, alleviate or ameliorate symptoms of the disease or prolong the survival of the subject being treated, which may be a human or non-human animal. Determination of a therapeutically effective amount is within the skill of the person skilled in the art.

The therapeutically effective amount or dosage of a compound according to this invention can vary within wide limits and may be determined in a manner known in the relevant art. The dosage can vary within wide limits and will, of course, have to be adjusted to the individual requirements in each particular case.

The inulin may be provided as such or in form of a composition, particularly a pharmaceutical composition. Said compositions may comprise additional medicinal agents, pharmaceutical agents, carriers, buffers, adjuvants, dispersing agents, diluents, and the like depending on the intended use and application.

Broadly, the present invention provides an oral nutritional composition, for oral consumption, wherein the nutritional composition includes the compounds of the present invention.

The compounds of the present invention and the pharmaceutical compositions containing said compounds, may be administered orally, and thus be formulated in a form suitable for oral administration, i.e. as a solid or a liquid preparation. Suitable solid oral formulations include tablets, capsules, pills, granules, pellets and the like. Suitable liquid oral formulations include solutions, suspensions, dispersions, emulsions, oils and the like. If formulated in form of a capsule, the compositions of the present invention comprise, in addition to the active compound and the inert carrier or diluent, a hard gelating capsule.

Examples of suitable pharmaceutical carriers, excipients and/or diluents are well known in the art and include, but are not limited to, a gum, a starch (e.g. corn starch, pregeletanized starch), a sugar (e.g., lactose, mannitol, sucrose, dextrose), a cellulosic material (e.g. microcrystalline cellulose), an acrylate (e.g. polymethylacrylate), calcium carbonate, magnesium oxide, talc, or mixtures thereof.

If the nutritional compositions are formulated to be administered orally, the compositions may be a liquid oral nutritional supplement (e.g., incomplete feeding) or a complete feeding. In this manner, the nutritional compositions may be administered in any known form including, for example, tablets, capsules, liquids, chewables, soft gels, sachets, powders, syrups, liquid suspensions, emulsions and solutions in convenient dosage forms.

"A nutritional composition may be a food product intended for human consumption, for example, a beverage, a drink, a bar, a snack, an ice cream, a dairy product, for example a chilled or a shelf-stable dairy product, a fermented dairy product, a drink, for example a milk-based drink, an infant formula, a growing-up milk, a confectionery product, a chocolate, a cereal product such as a breakfast cereal, a sauce, a soup, an instant drink, a frozen product intended for consumption after heating in a microwave or an oven, a ready-to-eat product, a fast food or a nutritional formula.

A nutritional formula encompasses any nutritionally complete or supplementary formulation (a nutritional supplement, for example). As used herein, "nutritionally complete" are preferably nutritional products that contain sufficient types and levels of macronutrients (protein, fats and carbohydrates) and micronutrients to be sufficient to be a sole source of nutrition for the subject to which it is being administered to. Patients can receive 100% of their nutritional requirements from such complete nutritional compositions. According to one embodiment, the nutritional formula is a supplementary formulation providing supplementary nutrition. A "supplementary formula" may not be nutritionally complete, but preferably contains specific nutrients that are supportive, for example in combination with physical exercise, with further of the beneficial effects of the invention, and/or which address specific or additional needs of the subject.

The nutritional formula may be a generally applicable nutritional formula, for example adapted to subjects of a specific age, for example a formula for children, but it may also be a formula for elderly patients, for intensive care patients, or a specially adapted formula for patients suffering from a specific disease, for example. Any nutritional formula may be reconstitutable, that is, present in a substantially dried, for example powdered form, or ready-to-drink, in the form of liquid formulas, for example .

Further details are enclosed in WO2012022947, which is incorporated herein by reference.

The pharmaceutical compositions provided herein may also be administered as controlled-release compositions, i.e. compositions in which the active ingredient is released over a period of time after administration. Controlled- or sustained-release compositions include formulation in lipophilic depots (e.g. fatty acids, waxes, oils). In another embodiment, the composition is an immediate-release composition, i.e. a composition in which all the active ingredient is released immediately after administration.

Further, the compound of formula (III) according to the invention and as described herein in the various embodiments may or a composition comprising said compound may be administered admixed to food, functional food, drinks, medicinal food.

The compounds of the present invention and the pharmaceutical compositions containing said compounds, may be administered orally, and thus be formulated in a form suitable for oral administration, i.e. as a solid or a liquid preparation. Suitable solid oral formulations include tablets, capsules, pills, granules, pellets and the like. Suitable liquid oral formulations include solutions, suspensions, dispersions, emulsions, oils and the like. If formulated in form of a capsule, the compositions of the present invention comprise, in addition to the active compound and the inert carrier or diluent, a hard gelating capsule.

The compounds of the present invention and the pharmaceutical compositions containing said compounds may be further administered intranasally, i.e. by inhalation and thus may be formulated in a form suitable for intranasal administration, i.e. as an aerosol or a liquid preparation.

The compounds of the present invention may also, for example, be formulated as suppositories, containing conventional suppository bases for use in human or veterinary medicine or as pessaries, for example, containing conventional pessary bases.

Examples of suitable pharmaceutical carriers, excipients and/or diluents are well known in the art and include, but are not limited to, a gum, a starch (e.g. corn starch, pregeletanized starch), a sugar (e.g., lactose, mannitol, sucrose, dextrose), a cellulosic material (e.g. microcrystalline cellulose), an acrylate (e.g. polymethylacrylate), calcium carbonate, magnesium oxide, talc, or mixtures thereof.

Pharmaceutically acceptable carriers for liquid formulations are aqueous or non-aqueous solutions, suspensions, emulsions or oils. Examples of non-aqueous solvents are propylene glycol, polyethylene glycol, and injectable organic esters such as ethyl oleate. Examples of oils are those of animal, vegetable, or synthetic origin, for example, peanut oil, soybean oil, olive oil, sunflower oil, fish-liver oil, another marine oil, or a lipid from milk or eggs.

Aqueous carriers include water, alcoholic/aqueous solutions, emulsions or suspensions, including saline and buffered media such as phosphate buffered saline solutions, water, emulsions, such as oil/water emulsions, various types of wetting agents, sterile solutions etc. Compositions comprising such carriers can be formulated by well known conventional methods. Suitable carriers may comprise any material which, when combined with the biologically active compound of the invention, retains the biological activity.

Preparations for parenteral administration may include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles may include sodium chloride solution, Ringer's dextrose, dextrose and sodium chloride, lactated Ringer's, or fixed oils. Intravenous vehicles may include fluid and nutrient replenishes, electrolyte replenishers (such as those based on Ringer's dextrose), and the like. Preservatives and other additives may also be present including, for example, antimicrobials, anti-oxidants, chelating agents, and inert gases and the like. In addition, the pharmaceutical composition of the present invention might comprise proteinaceous carriers, like, e.g., serum albumin or immunoglobulin, preferably of human origin.

The compounds of the present invention and as described herein in the various embodiments and the pharmaceutical compositions containing said compounds may be administered topically to body surfaces and thus be formulated in a form suitable for topical administration. Suitable topical formulations include gels, ointments, creams, lotions, drops and the like. For topical administration, the compound of formula (I) is prepared and applied as a solution, suspension, or emulsion in a physiologically acceptable diluent with or without a pharmaceutical carrier.

The pharmaceutical compositions provided herein may also be administered as controlled-release compositions, i.e. compositions in which the active ingredient is released over a period of time after administration. Controlled- or sustained-release compositions include formulation in lipophilic depots (e.g. fatty acids, waxes, oils). In another embodiment, the composition is an immediate-release composition, i.e. a composition in which all the active ingredient is released immediately after administration.

Further, the compound of formula (III) according to the invention and as described herein in the various embodiments may or a composition comprising said compound may be administered admixed to food, functional food, drinks, medicinal food.

Further examples for suitable formulations are provided in WO 2006/085983, the entire contents of which are incorporated by reference herein. For example, the polyfructans of the present invention may be provided as liposomal formulations. The technology for forming liposomal suspensions is well known in the art. When the adjuvant is an aqueous-soluble salt, using conventional liposome technology, the same can be incorporated into lipid vesicles. The lipid layer employed can be of any conventional composition and can either contain cholesterol or can be cholesterol-free. The liposomes can be reduced in size, as through the use of standard sonication and homogenization techniques. Liposomal formulations containing the adjuvant can be lyophilized, alone or with immunogen, to produce a lyophilizate which can be reconstituted with a pharmaceutically acceptable carrier, such as water, to regenerate a liposomal suspension. These pharmaceutical compositions can be administered to the subject at a suitable dose. The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently.

The polyfructan, in particular inulin of formula (III) and as described herein in the various embodiments may used in human and veterinary medicine for treating humans and animals, including avians, non-human primates, dogs, cats, pigs, goats, sheep, cattle, horses, mice, rats and rabbits.

These pharmaceutical compositions can be administered to the subject at a suitable dose. The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently.

The pharmaceutical composition of the invention as described herein in the various embodiments may used in human and veterinary medicine for treating humans and animals, including avians, non-human primates, dogs, cats, pigs, goats, sheep, cattle, horses, mice, rats and rabbits.

Suitable dosages of the inulin according to the invention and as described herein in the various embodiments will vary depending upon the condition, age and species of the subject, and can be readily determined by those skilled in the art. The total daily dosages of the compound of formula (III) employed in both veterinary and human medicine will suitably be in the range 0,01-2000 mg/kg body-weight, preferably from 0,1-1000 mg/kg body-weight, preferably from 1-100 mg/kg and these may be administered as single or divided doses, and in addition, the upper limit can also be exceeded when this is found to be indicated. Such dosage will be adjusted to the individual requirements in each particular case including the specific compound(s) being administered, the route of administration, the condition being treated, as well as the patient being treated. However, the compounds can also be administered as depot preparations (implants, slow-release formulations, etc.) weekly, monthly or at even longer intervals. In such cases the dosage will be much higher than the daily one and has to be adapted to the administration form, the body weight and the concrete indication. The appropriate dosage can be determined by conducting conventional model tests, preferably animal models. In general, in the case of oral or parenteral administration to adult humans weighing approximately 70 kg, a daily dosage of about 10 mg to about 10.000 mg, preferably from about 200 mg to about 1.000 mg, should be appropriate, although the upper limit may be exceeded when indicated. The daily dosage can be administered as a single dose or in divided doses, or for parenteral administration; it may be given as continuous infusion.

An effective dose of active ingredient(s) depends at least on the nature of the condition being treated, toxicity, whether the compound(s) is being used prophylactically (lower doses) or against an active infection or condition, the method of delivery, and the pharmaceutical formulation, and will be determined by the clinician using conventional dose escalation studies. It can be expected to be from about 0.05 to about 30 mg/kg body weight per day. For example, for topical delivery the daily candidate dose for an adult human of approximately 70 kg body weight will range from about 1 mg to about 500 mg, generally between about 5 mg and about 40 mg, and may take the form of single or multiple doses or administration sites.

If used as an adjuvant, the polyfructan, preferably inulin, of the invention and as described herein in the various embodiments and/or the immunogen may be given in form of a single or multiple (i.e., booster) dosage.

The immunogen and adjuvant can be co-administered concurrently (e.g., within hours of each other) in the same or different composition and, in the latter case, by the same or different route. Alternatively, the adjuvant can be administered prior to or after administration of the immunogen (e.g., about 6, 12, 24, 36, 48, 72, 96 or 120 hours or more before or after administration of the immunogen).

Further, if used as an adjuvant, the polyfructan, preferably inulin of the invention and as described herein in the various embodiments may administered mixed with immunogen, such as viral antigens, to enhance the immune response elicited against these antigens or alternatively the compound may be chemically coupled to the immunogen directly or in the case of particles (e.g. nanoparticles or virus-like particles (VLP)) the compound could be bound to the surface or encapsulated within said particles.

Furthermore, it is envisaged that the pharmaceutical composition of the invention might comprise further biologically active agents, depending on the intended use of the pharmaceutical composition. These further biologically active agents may be e.g. antibodies, antibody fragments, hormones, growth factors, enzymes, binding molecules, cytokines, chemokines, nucleic acid molecules and drugs. In a preferred embodiment, the pharmaceutical composition of the present invention is to be co-administered with other known immunosuppressive drug or treatments. Such immunosuppressive drugs may be selected from the group consisting of glucocorticoids, cytostatics such as methotrexate, myophenolate or azathioprine, antibodies such as T cell receptor directed antibodies or IL-4 receptor directed antibodies and drugs acting on immunophilins such as cyclosporine, tacrolimus, sirolimus and the like.

Unless otherwise defined, all technical and scientific terms used herein have the same meaning as commonly understood by one of ordinary skill in the art to which this invention pertains. Although methods and materials similar or equivalent to those described herein can be used in the practice or testing of the present invention, suitable methods and materials are described below. In case of conflict, the present specification, including definitions, will control. In addition, the materials, methods, and examples are illustrative only and not intended to be limiting.

The general methods and techniques described herein may be performed according to conventional methods well known in the art and as described in various general and more specific references that are cited and discussed throughout the present specification unless otherwise indicated. See, e.g., Sambrook et al., Molecular Cloning: A Laboratory Manual, 2d ed., Cold Spring Harbor Laboratory Press, Cold Spring Harbor, N.Y. (1989) and Ausubel et al., Current Protocols in Molecular Biology, Greene Publishing Associates (1992), and Harlow and Lane Antibodies: A Laboratory Manual, Cold Spring Harbor Laboratory Press, Cold Spring Harbor, N.Y. (1990).

While aspects of the invention are illustrated and described in detail in the drawings and foregoing description, such illustration and description are to be considered illustrative or exemplary and not restrictive. It will be understood that changes and modifications may be made by those of ordinary skill within the scope and spirit of the following claims. In particular, the present invention covers further embodiments with any combination of features from different embodiments described above and below.

The invention also covers all further features shown in the figures individually, although they may not have been described in the previous or following description. Also, single alternatives of the embodiments described in the figures and the description and single alternatives of features thereof can be disclaimed from the subject matter of the other aspect of the invention.

Furthermore, in the claims the word "comprising" does not exclude other elements or steps, and the indefinite article "a" or "an" does not exclude a plurality. A single unit may fulfill the functions of several features recited in the claims. The terms "essentially", "about", "approximately" and the like in connection with an attribute or a value particularly also define exactly the attribute or exactly the value, respectively. Any reference signs in the claims should not be construed as limiting the scope.

The patent or application file contains at least one drawing executed in color. Copies of this patent or patent application publication with color drawing(s) will be provided by the Office upon request and payment of the necessary fee.

The present invention is also illustrated in some aspects by the following figures.

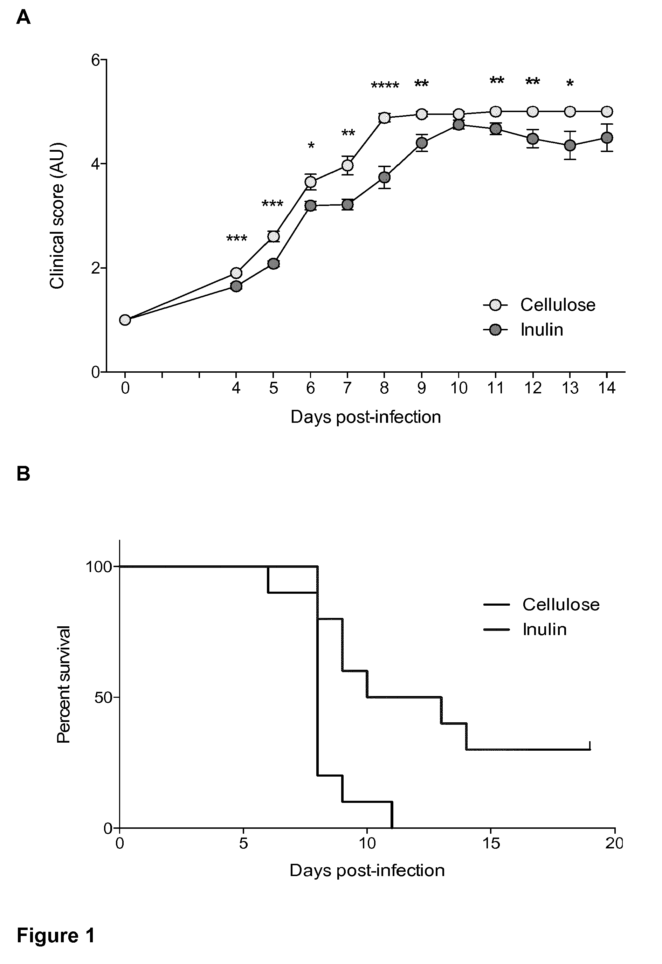

- **Figure 1**: **Clinical score of mice fed with inulin or cellulose.** The figure 1A shows the clinical score (AU) of mice fed with either a diet containing 30% cellulose or 30% inulin, respectively, over two weeks post-infection with influenza. Figure 1B shows the survival percentage of these mice for a period of three weeks post-infection.

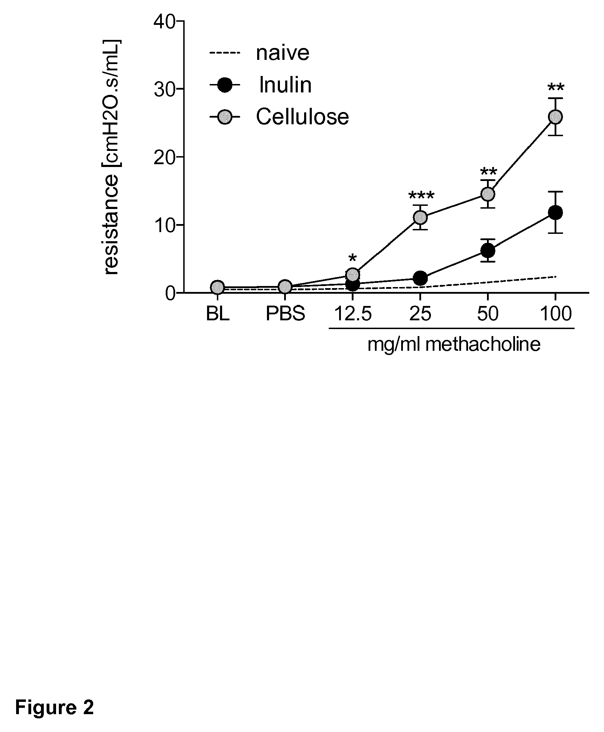

- **Figure 2**: **Airway resistance of mice**. Figure 2 shows airway resistance of mice fed either a diet containing 30% cellulose or 30% inulin.

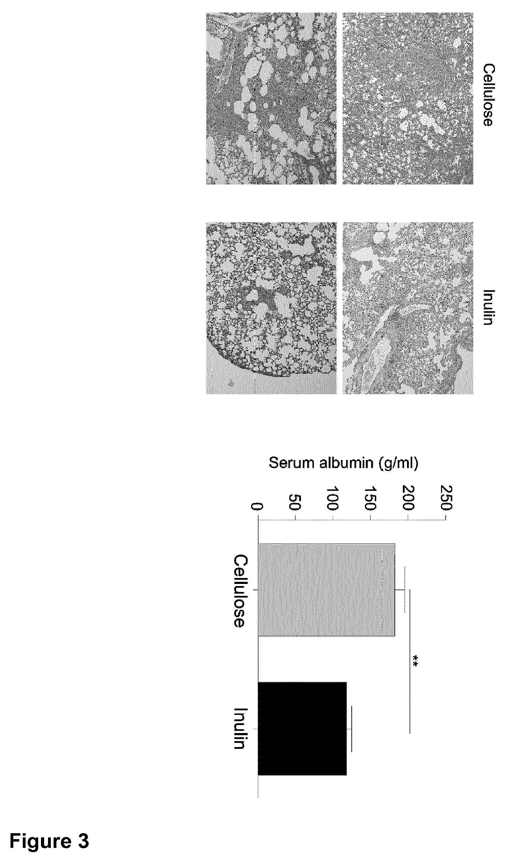

- **Figure 3**: **Lung pathology of mice.** Figure 3 shows lung pathology of mice fed either a diet containing 30% cellulose or 30% inulin as a tissue stain and expressed as serum albumin content.

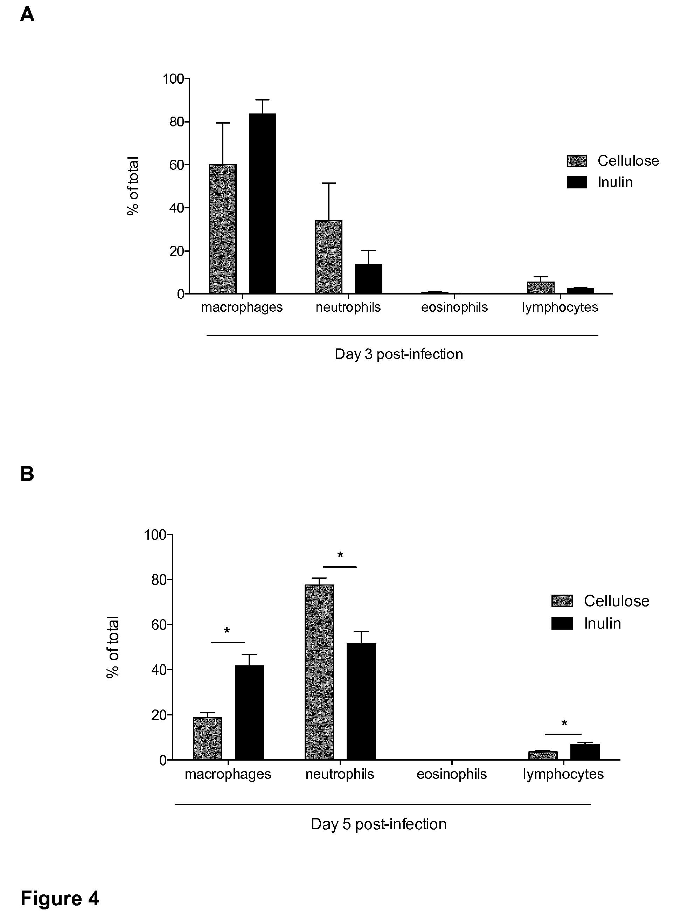

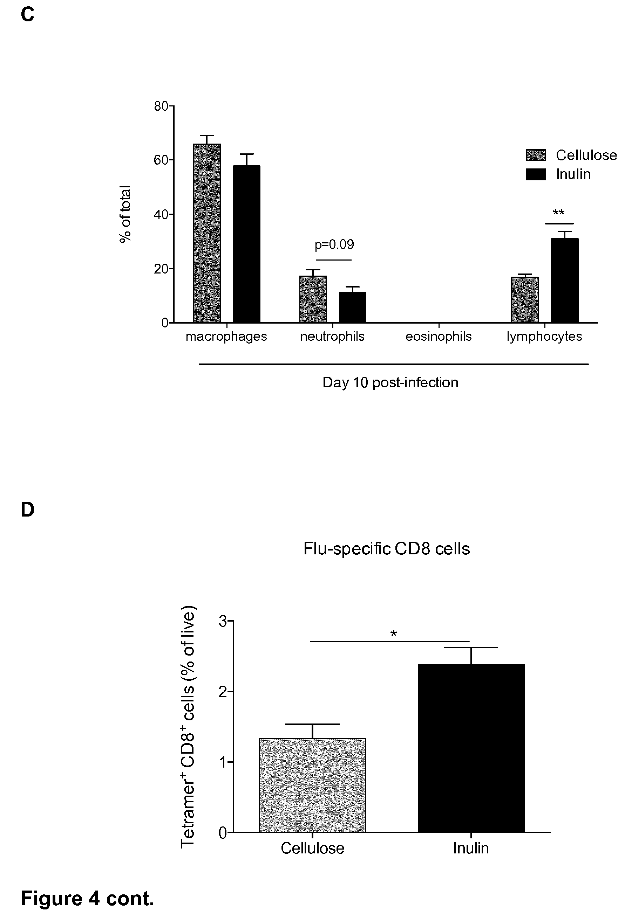

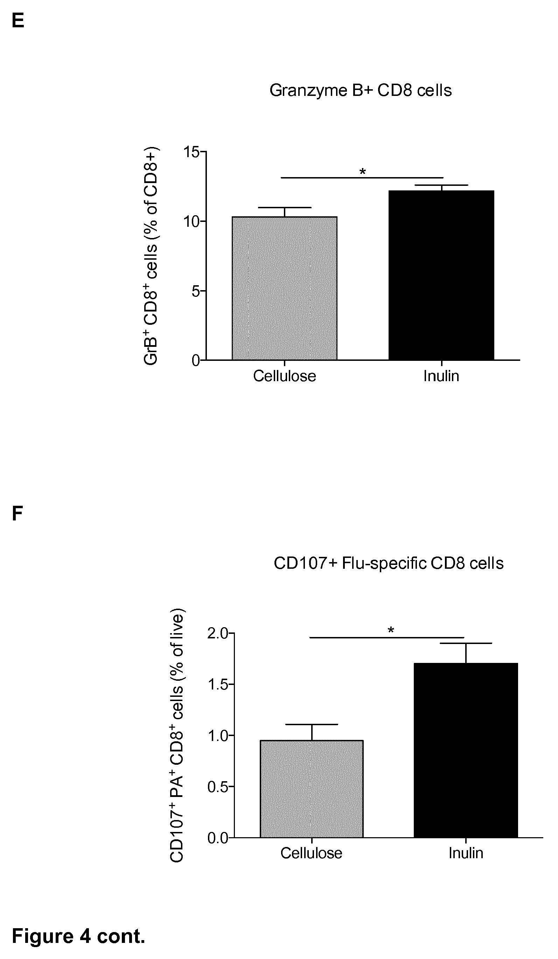

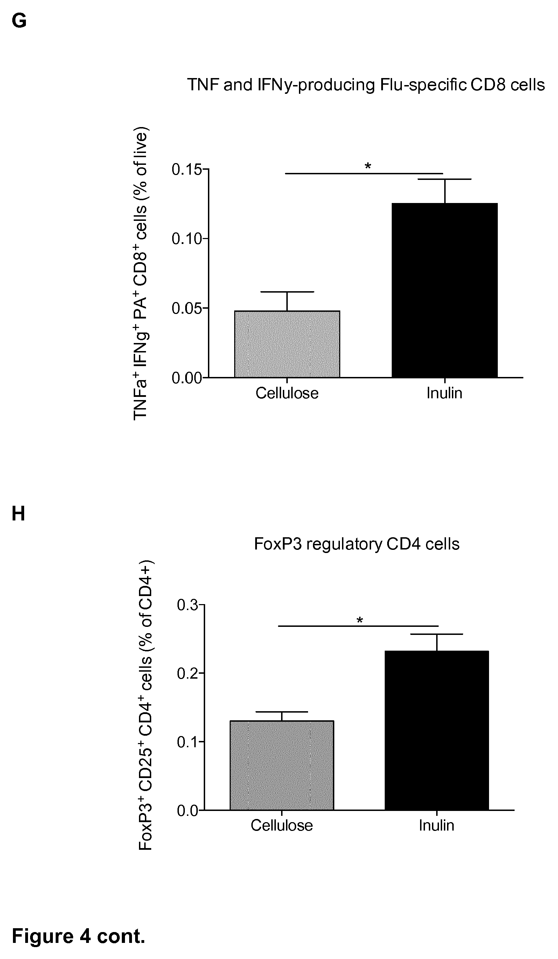

- **Figure 4**: **Protection against cell infiltration following infection**. Figure 4A, B and C show infiltration of different cell types in airway cells at day 3 post-infection, day 5 post-infection and day 10 post-infection. Figure 4D, E, F, G and H show ratios of different T-cells recruited in mice fed a high diet of inulin or cellulose, respectively.

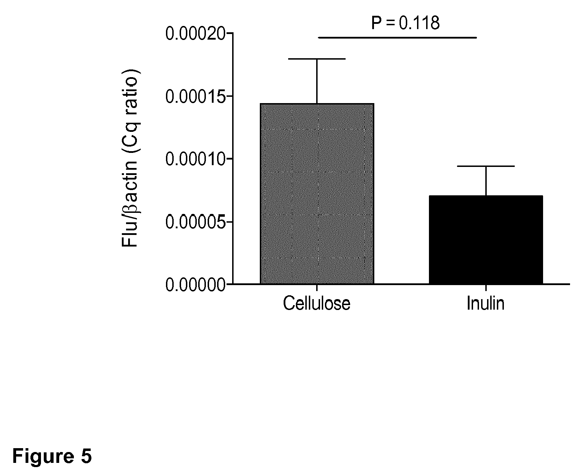

- **Figure 5**: **Protection against influenza virus**. Lung fed a diet high in inulin or cellulose, respectively, were tested for the presence of influenza virus gene expression in airway cells.

Aspects of the present invention are additionally described by way of the following illustrative non-limiting examples that provide a better understanding of embodiments of the present invention and of its many advantages. The following examples are included to demonstrate preferred embodiments of the invention. It should be appreciated by those of skill in the art that the techniques disclosed in the examples which follow represent techniques used in the present invention to function well in the practice of the invention, and thus can be considered to constitute preferred modes for its practice. However, those of skill in the art should appreciate, in light of the present disclosure, that many changes can be made in the specific embodiments which are disclosed and still obtain a like or similar result without departing from the spirit and scope of the invention.

A number of documents including patent applications, manufacturer's manuals and scientific publications are cited herein. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

### Example 1 Determining whether mice fed a diet high in Inulin were protected against lethal infection with influenza virus.

Protocol: BALB/c mice were fed either a diet containing 30% purified cellulose (Vitacel LC 200, Rettenmaier & Söhne) (Cellulose group) or 30% purified inulin (Fibruline instant, Cosucra) (Inulin group) as the fiber content. 8 week-old mice on these diets were then infected with 1122 PFU of Influenza strain A/Puerto Rico/8/1934 H1N1 (PR8) intranasally in a final volume of 50 µl. Mice were monitored on the indicated days to assess clinical symptoms and mortality. The clinical score was determined by: 1 point for a healthy mouse; 2 points for a mouse showing signs of malaise, including slight piloerection, slightly changed gait and increased ambulation; 3 points for a mouse showing signs of strong piloerection, constricted abdomen, changed gait, periods of inactivity; 4 points for a mouse with enhanced characteristics of the previous group, but showing little activity and becoming moribund; 4.5 points for a mouse with enhanced characteristics of the previous group, but showing little activity, is moribund and due to ethics restrictions needs to be removed from the study and is scored as 5 on the following measurement time point. 5 points for a dead mouse. A Student's t-test was performed at each time point between the two groups, with '*' = p<0.05, '**' = p<0.01, '***' p<0.005, '****' = p<0.001. The results show that mice infected with a lethal dose of virus have a greater rate of survival if they have eaten a diet containing inulin. Moreover, the mice that have eaten an inulin diet have a reduced symptoms of disease as noted by less malaise, more mobility and overall a better wellbeing.

Conclusion: Mice fed a diet high in the fermentable fiber, inulin, are protected against morbidity and mortality as compared to mice on a control diet high in the non-fermentable fiber, cellulose.

### Example 2 Determining whether mice fed a diet high in Inulin were protected against lung dysfunction following infection with influenza virus.

Protocol: BALB/c mice were fed either a diet containing 30% cellulose (Cellulose group) or 30% inulin (Inulin group) as the fiber content. Eight week old mice on these diets were then infected with 1122 PFU of Influenza strain A/Puerto Rico/8/1934 H1N1 (PR8) intranasally in a final volume of 50 µl. On day 7 post infection airway resistance was measured by airflow perturbations using the FlexiVent invasive airway mechanics system from Scireq. Mice were anesthetized by administering 100 mg/kg ketamine (Ketasol-100, Graeub) intramuscularly and of 50 mg/kg pentobarbital (Esconarkon, Streuli Pharma) intraperitoneally. Subsequently, mice were tracheotomized and mechanically ventilated at a rate of 450 breaths/min and a tidal volume of 10 ml/kg bodyweight. A Student's t-test was performed at each time point between the two groups, with '*' = p<0.05, '**' = p<0.01, '***' p<0.005. The result gives a direct measurement of how well the lungs are functioning during an influenza infection, and shows that mice eating a diet including inulin have better lung function (ease of breathing) than mice that have not eaten inulin.

Conclusion: Mice fed a diet high in the fermentable fiber, inulin, are protected against lung dysfunction as compared to mice on a control diet high in the non-fermentable fiber, cellulose.

### Example 3 Determining whether mice fed a diet high in Inulin were protected against lung pathology following infection with influenza virus.

### Protocol:

BALB/c mice were fed either a diet containing 30% cellulose (Cellulose group) or 30% inulin (Inulin group) as the fiber content. Eight week old mice on these diets were then infected with 1122 PFU of Influenza strain A/Puerto Rico/8/1934 H1N1 (PR8) intranasally in a final volume of 50 µl. On day 7 post infection mice were euthanized by intra-peritoneal administration of 200 mg/kg of sodium pentobarbital (Esconarkon, Streuli Pharma AG, Uznach, catalogue number V102013). Lungs were removed and immediately stored in 4% formalin followed by standard histological processing and staining with hematoxylin and eosin stain. The tissue sections were examined and photographed. Serum albumin was measured in broncheoalveolar lavage fluid (BALF) using an ELISA kit (ICL, catalogue number E-90AL) and following manufacturer's instructions. A Student's t-test was performed at each time point between the two groups, with '*' = p<0.05, '**' = p<0.01, '***' p<0.005. These data show that mice that had eaten inulin had a healthier lung following influenza infection, in particular, this is shown by the reduced number of inflammatory cells that have entered the lung tissue. Moreover, the protective effect of inulin was also seen with the reduced serum albumin in the BALF of mice; this shows that there is less damage to the vasculature of the lung in mice that had eaten inulin.

Conclusion: Mice fed a diet high in the fermentable fiber, inulin, are protected against lung pathology and vascular leakage caused by influenza virus, as compared to mice on a control diet high in the non-fermentable fiber, cellulose.

### Example 4 Determining whether mice fed a diet high in Inulin were protected against inflammatory cell infiltration into the airways following infection with influenza virus.

**Protocol:** BALB/c mice were fed either a diet containing 30% cellulose (Cellulose group) or 30% inulin (Inulin group) as the fiber content. Eight week old mice on these diets were then infected with 100 PFU of Influenza strain A/Puerto Rico/8/1934 H1 N1 (PR8) intranasally in 50 µl final volume. On day 3, 5 and 10 post infection mice were euthanized by intra-peritoneal administration of sodium pentobarbital (Esconarkon, Streuli Pharma AG, Uznach, catalogue number: V102013). A broncheoalveolar lavage (BAL) was performed by instilling 0.5 ml of phosphate buffered saline (PBS) into the airways. BAL fluid was centrifuged at 1200 rpm in a refrigerated table-top centrifuge (Centriguge 5415R, Eppendorf) for 5 minutes, and the cellular pellet was resuspended in 1 ml of PBS supplemented with 0.2% bovine serum albumin (BSA). Total cells were counted using a Z2 Coulter counter (Beckman Coulter) and 50000 cells were then cytospun onto glass slides (Cytospin 3 centrifuge, Shandon), stained with Diffquik straining (Siemens, catalogue number: 130832) for morphological analysis. Once stained, cells were examined, counted (200 cells counted twice) and based upon standard morphological and cytochemical criteria were determined to be eosinophils, macrophages, neutrophils or lymphocytes. Remaining cells from the BALF were stained for 20 minutes at 4°C with the following antibodies: CD4-PerCP-Cy5.5 (catalogue number: 100434), CD8-PE-Cy7 (catalogue number: 100722), CD107-APC-Cy7 (catalogue number: 121616), Granzyme B-FITC (catalogue number: 515405), FoxP3-APC (catalogue number: 126408). All antibodies are from Biolegend. Influenza-specific CD8 cells were identified by utilizing a PE-labeled tetramer against the Influenza Nucleoprotein (NP) sequence TYQRTRALV (TCMetrix). Cells were then fixed 15 minutes at 4°C using BD FACS Lysing Solution (Becton Dickinson, catalogue number: 349202) before resuspension in PBS supplemented with 0.2% BSA. Samples were acquired by flow cytometry using a BD LSR II followed by analysis with Flowjo (version10.0.8). A Student's t-test was performed at each time point between the two groups, with '*' = p<0.05, '**' = p<0.01, '***' p<0.005. This data shows that mice that had eaten inulin had a reduced number of inflammatory granulocytes (neutrophils) in their airways following influenza infection. This is important because too many neutrophils in the airways can cause damage to the lung tissue, thus eating the inulin diet protected the mice against too much tissue damage. In addition, this data shows that the T cells which are critical for fighting the virus are increased in cell number in mice that had eaten inulin, thus helping to clear the virus from the lungs improving the health of the mice.

Conclusion: Mice fed a diet high in the fermentable fiber, inulin, are protected against the infiltration of neutrophils into the airways following influenza virus infection, as compared to mice on a control diet high in the non-fermentable fiber, cellulose. Mice fed a diet high in the fermentable fiber, inulin, exhibit increased recruitment of lymphocytes, of which a higher proportion of cells are highly activated cytotoxic T cells specific to the influenza virus and T regulatory cells, into the airways following influenza virus infection, as compared to mice on a control diet high in the non-fermentable fiber, cellulose.

### Example 5 Determining whether mice fed a diet high in Inulin were protected against influenza virus load in the lung following infection.

**Protocol**: BALB/c mice were fed either a diet containing 30% cellulose (Cellulose group) or 30% inulin (Inulin group) as the fiber content. Eight week old mice on these diets were then infected with 100 PFU of Influenza strain A/Puerto Rico/8/1934 H1 N1 (PR8) intranasally in 50 µl final volume. On day 10 post infection mice were euthanized by intra-peritoneal administration of 200 mg/kg of sodium pentobarbital (Esconarkon, Streuli Pharma AG, Uznach, catalogue number: V102013). Lung lobes were carefully isolated and placed in 2 ml Eppendorf tubes containing 1 ml of TRI-Reagent (Molecular Research Centre Inc., catalogue number: TR 118) and snap frozen in dry ice. Samples were then stored at -80°C until processing.

### RNA extraction

Lung lobes preserved in TRI-Reagent, were slowly thawed at room temperature. Sterile, stainless steel beads (Qiagen. Catalogue Number: 69989) were placed in each sample tube and lungs were homogenised using a TissueLyser (Qiagen. Catalogue Number: 85220), at a frequency of 25Hz for 3 minutes. RNA extraction using TRI-Reagent (Molecular Research Centre Inc. Catalogue number: TR 118) was completed following manufacturer's instructions. RNA concentration in each sample was measured using a Thermo Scientific NanoDrop^{™} 1000 Spectrophotometer (Thermo Fisher Scientific).

### PCR amplification

Samples were amplified in a one-step RT-PCR at a final reaction volume of 10 µl, containing 5µl of iTaq universal SYBR Green reaction mix (BIO-RAD Laboratories, Catalogue number 172-5150), 0.125µl of iScript reverse transcriptase (BIO-RAD. Catalogue number: 172-5150), 0.5µl of Influenza PR8 Matrix protein forward and reverse primers (5_-GGACTGCAGCGTAGACGCTT-3_ and 5_-CATCCTGTATATGAGGCCCAT-3) or Beta actin (5 -GATCAAGATCATTGCTCCTCCTGA-3_ and 5_-CAGCTCAGTAACAGTCCGCC- 3_), 0.375µl of RNAse free water (BIO-RAD. Catalogue number: 172-5150), and 4µl of RNA at 125ng/µl. Thermo-cycling was performed in a CFX-96 real-time PCR system (BIO-RAD) using the following protocol: Reverse transcription reaction: 10 min at 50°C, then polymerase activation and DNA denaturation: 1 min at 95°C, then denaturation: 95°C for 10 sec, then annealing/ extension + plate read: 30sec at 60°C, for total of 40 cycles.

This data shows that mice which had eaten inulin had less influenza virus in their lungs, which shows that the immune response against the virus was better in those mice, compared to mice that had not eaten inulin.

**Conclusion:** Mice fed a diet high in the fermentable fiber, inulin, are protected against influenza virus infection, as compared to mice on a control diet high in the non-fermentable fiber, cellulose.

## Claims

1. A polyfructan for use in treating and/or attenuating and/or preventing viral infections, bacterial infections and/or inflammation.

2. The polyfructan for use according to claim 1, wherein the polyfructan comprises a β-2,1-linked chain of fructose residues.

3. The polyfructan for use according to claim 2, wherein the fructose residue at the 2-OH terminus of said chain is bound, preferably via a β**-**2,1-linkage, to a glucose residue.

4. The polyfructan for use according to any one of claims 2 or 3, wherein the polyfructan is a polydispersed mixture of molecules of differing chain length.

5. The polyfructan for use according to any one of claims 2 to 4, wherein the polyfructan is inulin.

6. The polyfructan for use according to claim 5, wherein the inulin has a degree of polymerization of between about 2 to about 60, preferably between about 10 to about 60.

7. The polyfructan for use according to claims 5 or 6, wherein the inulin is derived from plants.

8. The polyfructan for use according to claim 7, wherein the inulin is derived from the roots of the plant.

9. The polyfructan for use according to claims 7 or 8, wherein the plant is of the genus *Cynara,* preferably *Cynara cardunculus* or *Cynara scolymus, Cichorium,* preferably *Cichorium intybus, Helianthus,* preferably *Helianthus tuberosus, Dahlia, Agave,* preferably *Agave tequilana,* or *Taraxacum.*

10. A composition comprising the polyfructan for use according to any one of claims 1 to 9.

11. The composition of claim 10, wherein the polyfructan content is between about 5 to about 60, preferably about 10 to about 50, about 20 to about 40%, or about 30% (w/w).

12. The composition of claims 10 or 11, further comprising a short-chain fatty acid.

13. The composition of claim 12, wherein the short-chain fatty acid is butyric acid, propionic acid or acetic acid.

14. The polyfructan for use according to any one of claims 1 to 19 or the composition for use according to any one of claims 10 to 13, wherein the polyfructan or the composition, respectively, is to be administered orally, sublingually, inhaled, topically, intranasally, parenterally, intravenously, intramuscularly or subcutaneously.


SEQUENCE LISTING

<110> Proponent Biotech GmbH

<120> Uses of polyfructans

<130> Y3168 EP BS

<140> EP16 15 6138.6
<141> 2016-02-17

<160> 4

<170> BiSSAP 1.3

<210> 1
<211> 20
<212> DNA
<213> Artificial Sequence

<220>
<223> PR8 Matrix protein forward primer

<400> 1
ggactgcagc gtagacgctt                                                    20


<210> 2
<211> 21
<212> DNA
<213> Artificial Sequence

<220>
<223> PR8 Matrix protein reverse primer

<400> 2
catcctgtat atgaggccca t                                                  21


<210> 3
<211> 24
<212> DNA
<213> Artificial Sequence

<220>
<223> Beta actin forward primer

<400> 3
gatcaagatc attgctcctc ctga                                               24


<210> 4
<211> 20
<212> DNA
<213> Artificial Sequence

<220>
<223> Beta actin reverse primer

<400> 4
cagctcagta acagtccgcc                                                    20